(19) Europäisches Patentamt
European Patent Office
Office européen des brevets

(11) Numéro de publication : **0 385 882 B1**

(12) # FASCICULE DE BREVET EUROPEEN

(45) Date de publication du fascicule du brevet : **19.11.92 Bulletin 92/47**

(51) Int. Cl.$^5$ : **C07C 37/60,** C07C 39/08, C07C 43/23, B01J 21/06, C07C 41/26

(21) Numéro de dépôt : **90420055.7**

(22) Date de dépôt : **01.02.90**

(54) **Procédé d'hydroxylation de phénols et d'éthers de phénols.**

(30) Priorité : **28.02.89 FR 8902868**

(43) Date de publication de la demande : **05.09.90 Bulletin 90/36**

(45) Mention de la délivrance du brevet : **19.11.92 Bulletin 92/47**

(84) Etats contractants désignés : **AT BE CH DE DK ES FR GB GR IT LI LU NL SE**

(56) Documents cités :
**EP-A- 0 200 260**
**EP-A- 0 226 257**
**DE-A- 2 162 552**
**GB-A- 2 116 974**
**US-A- 3 110 653**
**CHEMISTRY LETTERS, no. 8, 1981, pages 1053-1056, The Chemical Society of Japan, Tokyo, JP; M. FUJIHIRA et al, "Heterogenousphotocatalytic oxidation of aromatic compounds on semiconductor materials: the photo-fenton type reaction"**

(73) Titulaire : **RHONE-POULENC CHIMIE**
**25, quai Paul Doumer**
**F-92408 Courbevoie Cédex (FR)**

(72) Inventeur : **Costantini, Michel**
**10 rue du Docteur Bonhomme**
**F-69003 Lyon (FR)**
Inventeur : **Garcin, Eric**
**72 rue Maurice Arnoux**
**F-92120 Montrouge (FR)**
Inventeur : **Gubelmann, Michel**
**39 boulevard des Belges**
**F-69006 Lyon (FR)**
Inventeur : **Popa, Jean-Michel**
**2 rue Roger Breton**
**F-93700 Drancy (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

EP 0 385 882 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'hydroxylation de phénols ou d'éthers de phénols par le peroxyde d'hydrogène.

L'hydroxylation du phénol ou de phénols substitués, par le peroxyde d'hydrogène, pour préparer des diphénols est une réaction connue.

Le brevet français n° 69-45467 publié sous le n° 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

Ainsi, la demande de brevet français n° 81-17023 (publiée sous le n° 2 489 816) préconise l'emploi de silicalite de titane comme catalyseur hétérogène de l'hydroxylation de composés aromatiques par le peroxyde d'hydrogène.

La fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

Il a également été proposé dans la demande de brevet européen EP-A-0 299 893 d'utiliser comme catalyseur des argiles pontées. Bien que des résultats intéressants aient ainsi été obtenus, les recherches concernant la catalyse hétérogène de l'hydroxylation des phénols ou éthers de phénols par le peroxyde d'hydrogène ont été poursuivies.

C'est ainsi que la présente invention a été effectuée.

L'invention consiste donc en un procédé d'hydroxylation de phénols ou éthers de phénols de formule générale (I) :

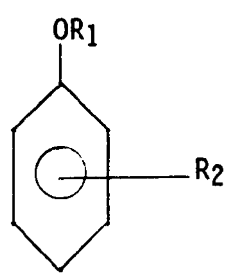

(I)

dans laquelle :

- $R_1$ représente un atome d'hydrogène, un groupement méthyle, un groupement éthyle ou un groupement phényle,
- $R_2$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de dioxyde de titane.

Il existe différentes variétés naturelles et artificielles de dioxyde de titane, qui peuvent être utilisées dans le cadre de l'invention.

Ainsi on peut mettre en oeuvre le dioxyde de titane anatase, le dioxyde de titane rutile et le dioxyde de titane brookite, bien que cette dernière forme soit rare car son domaine thermique de stabilité est étroit.

Le dioxyde de titane naturel peut être utilisé comme catalyseur, mais le plus fréquemment on utilise les dioxydes de titane obtenus artificiellement, qui se trouvent dans le commerce.

On peut également le préparer à partir des minerais tels que l'ilménite.

Le dioxyde de titane peut également être soumis à un traitement avant son emploi comme catalyseur dans le procédé d'hydroxylation de l'invention.

Ainsi on peut procéder à une calcination pendant une à plusieurs heures à une température de 300°C à

1000°C environ et de préférence de 300°C à 700°C. On peut également obtenir un dioxyde de titane de structure différente par exemple en faisant réagir du $TiO_2$ anatase avec du nitrate de potassium à haute température (supérieure à 800°C) pour former du titanate de potassium, en hydrolysant à chaud le titanate obtenu et en le calcinant à une température de 400 à 600°C environ.

Le dioxyde de titane peut être utilisé sous diverses formes : poudre ou produits mis en forme tels que granulés (par exemple des cylindres ou des trilobes), billes ou monolithes (blocs en forme de nid d'abeilles) qui sont obtenus par extrusion, moulage, compactage ou tout autre type de procédé connu.

En pratique, ce sont les formes de granulés, de billes et de monolithes qui présentent le plus d'avantages, tant sur le plan de l'efficacité, car les sites actifs du catalyseur sont plus facilement atteints par la diffusion des réactifs, que sur le plan de la commodité de la manipulation.

Les phénols et éthers de phénols qui sont utilisés de préférence dans le procédé de l'invention sont les composés de formule (I) dans laquelle $R_1$ représente un atome d'hydrogène, un groupement méthyle ou un groupement éthyle, et $R_2$ représente un atome d'hydrogène, un groupement méthyle, éthyle ou tertiobutyle, un groupement méthoxy ou éthoxy.

A titre d'exemples non limitatifs on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchol.

On note que le rapport pyrocatéchol/hydroquinone obtenu est le plus souvent supérieur à 2, ce qui comptetenu des importances relatives des débouchés de ces 2 diphénols est particulièrement intéressant.

On peut même obtenir des rapports pyrocatéchol/hydroquinone supérieurs à 3.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl-tertiobutyléther.

Le rapport molaire composé de formule I/peroxyde d'hydrogène est généralement de 25/1 à 3/1 et préférentiellement de 20/1 à 4/1.

La quantité d'oxyde de titane défini précédemment, que l'on peut mettre en oeuvre dans le présent procédé peut varier dans de très larges limites.

Lorsque l'on réalise le procédé en discontinu, le catalyseur peut représenter en poids par rapport au poids du composé de formule (I) engagé de 0,1 % à 20 %. De préférence, ce rapport pondéral sera compris entre 0,5 % et 10 %. Cependant si l'on réalise le procédé en continu, par exemple en faisant réagir un mélange de composé (I) et de solution de peroxyde d'hydrogène sur un lit fixe de catalyseur ou sur un monolithe, ces rapports catalyseur/composé (I) n'ont plus de sens et, à un instant donné, on pourra avoir un excès pondéral de catalyseur par rapport au composé (I).

Il est également possible d'effectuer la réaction d'hydroxylation du composé (I) dans un solvant du composé (I), qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitrile ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-méthylpyrrolidone.

La température à laquelle est conduite la réaction est généralement comprise entre 45° et 160°C sous pression atmosphérique. On peut également opérer à plus haute température et sous pression supérieure à la pression atmosphérique.

Les exemples qui suivent illustrent l'invention.

PREPARATION DES CATALYSEURS UTILISES DANS LES EXEMPLES CI-APRES

I - $TiO_2$ (I) B de SBE = 6 $m^2/g$

On fait réagir à 1000°C du nitrate de potassium et du dioxyde de titane anatase, afin de préparer du tétratitanate de potassium $K_2Ti_4O_9$.

Ce tétratitanate de potassium est ensuite hydrolysé à chaud, puis calciné à 500°C pour donner le $TiO_2$ (I) B ayant une surface spécifique BET de 6 $m^2/g$.

II - $TiO_2$ (II) B de SBE = 62 $m^2/g$

On fait réagir à 1000°C du nitrate de potassium et du dioxyde de titane anatase, afin de préparer du tétratitanate de potassium $K_2$ $Ti_4$ $O_9$.

Ce tétratitanate de potassium est ensuite hydrolysé à chaud, calciné à 500°C, puis broyé pour donner le $TiO_2$ (II) B ayant une surface spécifique BET de 62 $m^2/g$.

III - $TiO_2$ (III) RUTILE de SBE = 23 $m^2/g$

On hydrolyse l'oxychlorure de titane. La solution obtenue est lavée par décantation, filtrée, puis séchée en étuve à 120°C pendant 24 heures.

IV - $TiO_2$ (IV) ANATASE de SBE = 4 $m^2/g$

On calcine à 650°C le $TiO_2$ (I) B préparé en I. On obtient ainsi du $TiO_2$ (IV) ANATASE ayant une surface spécifique BET de 4 $m^2/g$.

V - $TiO_2$ (V) ANATASE de SBE = 208 $m^2/g$

On attaque de manière classique par l'acide sulfurique de l'ilménite. A la suspension d'oxyde de titane obtenue après hydrolyse et filtration et contenant des anions sulfates en quantité telle que le rapport en poids $SO_4/TiO_2$ soit égal à 0,08, on incorpore de l'hydroxyde de calcium, de manière à faire réagir 90 % des sulfates.

La suspension est ensuite atomisée.

On obtient le $TiO_2$ (V) ANATASE ayant une surface spécifique BET de 208 $m^2/g$.

VI - $TiO_2$ (VI) ANATASE de SBE = 212 $m^2/g$

On attaque de manière classique par l'acide sulfurique de l'ilménite. A la suspension d'oxyde de titane obtenue après hydrolyse et filtration et contenant des anions sulfates en quantité telle que le rapport en poids $SO_4/TiO_2$ est égal à 0,08, on incorpore de l'hydroxyde de calcium, de manière à pouvoir faire réagir 120 % des sulfates.

La suspension est ensuite atomisée.

On obtient le $TiO_2$ (VI) ANATASE ayant une surface spécifique BET de 212 $m^2/g$.

VII - $TiO_2$ (VII) ANATASE de SBE = 286 $m^2/g$

On attaque de manière classique par l'acide sulfurique de l'ilménite. La suspension d'oxyde de titane obtenue après hydrolyse et filtration est lavée par une solution d'ammoniaque 2N de telle sorte que le rapport en poids $SO_4/TiO_2$ soit égal à 0,06.

La suspension est ensuite atomisée.

On obtient le $TiO_2$ (VII) ANATASE ayant une surface spécifique BET de 286 $m^2/g$.

VIII - $TiO_2$ (VIII) ANATASE de SBE = 105 $m^2/g$

On attaque de manière classique par l'acide sulfurique de l'ilménite. La suspension d'oxyde de titane obtenue après hydrolyse et filtration est lavée par une solution d'ammoniaque 2N de telle sorte que le rapport en poids $SO_4/TiO_2$ soit égal à 0,0001.

La suspension est ensuite atomisée.

On calcine 3 heures à 400°C.

On obtient le $TiO_2$ (VIII) ANATASE ayant une surface spécifique BET de 105 $m^2/g$ et un taux d'ions sulfates en surface de 0,01 %.

IX - $TiO_2$ (IX) ANATASE de SBE = 237 $m^2/g$

On attaque de manière classique par l'acide sulfurique de l'ilménite. La suspension d'oxyde de titane obtenue après hydrolyse et filtration est lavée par une solution d'ammoniaque 2N de telle sorte que le rapport en poids $SO_4/TiO_2$ soit égal à 0,01.

La suspension est ensuite atomisée.

On obtient le $TiO_2$ (IX) ANATASE ayant une surface spécifique BET de 237 $m^2/g$.

X - $TiO_2$ (X) ANATASE

On attaque de manière classique par l'acide sulfurique de l'ilménite. A la suspension d'oxyde de titane obtenue après hydrolyse et filtration et contenant des anions sulfates en quantité telle que le rapport en poids $SO_4/TiO_2$ soit égal à 0,08, on incorpore de l'hydroxyde de calcium, de manière à faire réagir 90 % des sulfates.

La suspension est ensuite atomisée, puis calcinée 3 heures à 400°C.

On obtient le $TiO_2$ (X) ANATASE ayant un taux d'ions sulfates en surface de 7 à 8 % et une surface spécifique BET de 190 $m^2/g$.

EXEMPLES 1 à 13

Dans un réacteur en verre pyrex de 30 $cm^3$, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 9,4 g de phénol (0,10 mol)
- 0,25 g de dioxyde de titane.

On chauffe à 80°C sous agitation, puis on injecte une solution aqueuse de $H_2O_2$ à 70 % en poids par poids. (0,0050 mol de $H_2O_2$).

On laisse ensuite réagir pendant 2 heures 30 minutes.

Après filtration du catalyseur, on dose $H_2O_2$ non transformée par iodométrie et les diphénols par chromatographie liquide haute performance (CLHP).

On détermine ainsi pour chaque essai :

- le taux de transformation (TT) de $H_2O_2$ ;
- le rendement en pyrocatéchol par rapport à $H_2O_2$ transformée (RT/PC) ;
- le rendement en hydroquinone par rapport à $H_2O_2$ transformée (RT/HQ) ;
- le rendement total en diphénols.

Le catalyseur utilisé ainsi que les résultats obtenus dans chaque essai, sont rassemblés dans le tableau 1 ci-après.

| EXEMPLES | CATALYSEURS | TT % H2O2 | RT % PC | RT % HQ | RT % DIPHENOLS | PC/HQ |
|---|---|---|---|---|---|---|
| 1 | $TiO_2$ (I) B de SBE = 6 m2/g | 46,5 | 11,0 | 0,5 | 11,5 | 22 |
| 2 | $TiO_2$ (II) B de SBE = 62 m2/g calciné 3 heures à 400°C | 96,5 | 12,5 | 6,5 | 19,0 | 1,9 |
| 3 | $TiO_2$ (III) RUTILE de SBE = 23 m2/g | 97,5 | 9,5 | 4,5 | 14,0 | 2,1 |
| 4 | $TiO_2$ (IV) ANATASE de SBE = 4 m2/g calciné 3 heures à 400°C | 41,5 | 11,0 | 3,0 | 14,0 | 3,6 |
| 5 | $TiO_2$ (V) ANATASE de SBE = 208 m2/g | 99,0 | 9,5 | 2,5 | 12,0 | 3,8 |
| 6 | $TiO_2$ (V) ANATASE de SBE = 208 m2/g calciné 5 heures à 550°C | 97,5 | 33,0 | 8,0 | 41,0 | 4,1 |
| 7 | $TiO_2$ (VI) ANATASE de SBE = 212 m2/g calciné 5 heures à 550°C | 88,0 | 35,5 | 6,5 | 42,0 | 5,5 |

Tableau 1

SBE = surface spécifique BET

| EXEMPLES | CATALYSEURS | TT % H2O2 | RT % PC | RT % HQ | RT % DIPHENOLS | PC/HQ |
|---|---|---|---|---|---|---|
| 8 | TiO2 (VII) ANATASE de SBE = 286 m2/g | 98,5 | 15,0 | 5,5 | 20,5 | 2,7 |
| 9 | TiO2 (VII) ANATASE de SBE = 286 m2/g calciné 5 heures à 550°C | 99,5 | 29,0 | 9,5 | 38,5 | 3,0 |
| 10 | TiO2 (VIII) ANATASE de SBE = 105 m2/g calciné 3 heures à 400°C | 75,5 | 35,0 | 9,5 | 44,5 | 3,7 |
| 11 | TiO2 (IX) ANATASE de SBE = 237 m2/g | 97,0 | 15,5 | 2,0 | 17,5 | 7,7 |
| 12 | TiO2 (IX) ANATASE de SBE = 237 m2/g calciné 5 heures à 550°C | 98,0 | 21,5 | 7,5 | 29,0 | 2,9 |
| 13 | TiO2 (X) ANATASE de SBE = 190 m2/g calciné 3 heures à 400°C | 99,5 | 13,5 | 4,5 | 18,0 | 3,0 |

Tableau 1 (fin)

SBE = surface spécifique BET

EXEMPLES 14 à 17

Dans un réacteur en verre pyrex de 30 $cm^3$, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 4,7 g de phénol (0,05 mol)
- 0,25 g de $TiO_2$ (VIII) ANATASE de SBE 105 $m^2/g$, calciné 3 heures à 400°C
- 4,7 g de solvant (voir tableau 2 ci-après).

On chauffe à 100°C sous agitation, puis on injecte une solution aqueuse de $H_2O_2$ à 70 % en poids par poids. (0,0025 mol de $H_2O_2$).

On laisse ensuite réagir pendant 2 heures 30 minutes.

Après filtration du catalyseur, on dose $H_2O_2$ non transformée par iodométrie et les diphénols par chromatographie liquide haute performance (CLHP).

Les résultats obtenus sont rassemblés dans le tableau 2 ci-après.

| EXEMPLES | SOLVANTS | TT % H2O2 | RT % PC | RT % HQ | RT % diphénols | Rapport PC/HQ |
|---|---|---|---|---|---|---|
| 14 | Acide acétique | 90,0 | 31,5 | 10,0 | 41,5 | 3,1 |
| 15 | Acétonitrile | 91,5 | 37,0 | 5,5 | 42,5 | 6,7 |
| 16 | Méthylisobutyl cétone | 52,5 | 27,0 | 4,5 | 31,5 | 6,0 |
| 17 | Sulfolane | 96,0 | 33,5 | 8,5 | 42,0 | 3,9 |

TABLEAU 2

EXEMPLE 18

On répète l'exemple 10, en ajoutant aux charges initiales 0,412 g (0,2 mmol) de tétrahydrogénodiphosphate d'hydroxy-1 éthane.

Après addition de $H_2O_2$, on maintient l'essai 20 heures à 80°C.

On obtient les résultats suivants :

- TT de $H_2O_2$ : 85,5 %
- RT en pyrocatéchol : 40,5 %
- RT en hydroquinone : 25,0 %
- RT total en diphénols : 65,5 %
- Rapport PC/HQ : 1,6

EXEMPLE 19

Dans un réacteur en verre pyrex de 100 $cm^3$, muni d'une agitation centrale, d'un réfrigérant relié à un gazomètre, d'un système de chauffage régulé et d'un système d'injection, on charge après avoir préalablement purgé l'appareil à l'aide d'azote :

- 50,1 g de phénol (0,53 mol)
- 0,499 g de $TiO_2$ (IX) ANATASE, calciné 1 h 30 à 400°C, puis 2 heures à 550°C.

On chauffe à 80°C sous agitation, puis on injecte 0,335 g d'une solution aqueuse de $H_2O_2$ à 69,5 % en poids par poids. (6,85 mmol de $H_2O_2$).

On laisse ensuite réagir pendant 45 minutes à 80°C.

Après filtration du catalyseur, on dose $H_2O_2$ non transformée par iodométrie et les diphénols par chromatographie liquide haute performance (CLHP).

On obtient les résultats suivants :

- TT de $H_2O_2$ : 99,5 %
- RT en pyrocatéchol : 58,0 %
- RT en hydroquinone : 13,5 %
- RT total en diphénols : 71,5 %
- Rapport PC/HQ : 4,3

EXEMPLE 20

Dans un réacteur en verre pyrex de 100 $cm^3$, muni d'une agitation centrale, d'un réfrigérant relié à un gazomètre, d'un système de chauffage régulé et d'un système d'injection, on charge après avoir préalablement purgé l'appareil à l'aide d'azote :

- 47,0 g de phénol (0,5 mol)
- 0,5 g de $TiO_2$ (IX) ANATASE, calciné 1 h 30 à 400°C, puis 2 heures à 550°C.

On chauffe à 80°C sous agitation, puis on injecte 1,287 g d'une solution aqueuse de $H_2O_2$ à 70 % en poids par poids. (26,5 mmol de $H_2O_2$).

On laisse ensuite réagir pendant 10 minutes à 80°C.

Après filtration du catalyseur, on dose $H_2O_2$ non transformée par iodométrie et les diphénols par chromatographie liquide haute performance (CLHP).

On obtient les résultats suivants :

- TT de $H_2O_2$ : 93,5 %
- RT en pyrocatéchol : 40,0 %
- RT en hydroquinone : 7,0 %
- RT total en diphénols : 47,0 %
- Rapport PC/HQ : 5,9

EXEMPLE 21

Dans un réacteur en verre pyrex de 100 $cm^3$, muni d'une agitation centrale, d'un réfrigérant relié à un gazomètre, d'un système de chauffage régulé et d'un système d'injection, on charge après avoir préalablement purgé l'appareil à l'aide d'azote :

- 47,0 g de phénol (0,5 mol)
- 0,5 g de $TiO_2$ (IX) ANATASE, calciné 1 h 30 à 400°C, puis 2 heures à 550°C.

On chauffe à 110°C sous agitation, puis on injecte 1,287 g d'une solution aqueuse de $H_2O_2$ à 70 % en poids par poids. (26,5 mmol de $H_2O_2$).

On laisse ensuite réagir pendant 5 minutes à 110°C.

Après filtration du catalyseur, on dose $H_2O_2$ non transformée par iodométrie et les diphénols par chromatographie liquide haute performance (CLHP).

On obtient les résultats suivants :

- TT de $H_2O_2$ : 98,0 %
- RT en pyrocatéchol : 34,0 %
- RT en hydroquinone : 9,0 %
- RT total en diphénols : 43,0 %
- Rapport PC/HQ : 3,8

EXEMPLE 22

On répète l'exemple 21 mais en réalisant la réaction à 150°C.

On obtient les résultats suivants :

- TT de $H_2O_2$ : 100 %
- RT en pyrocatéchol : 22,0 %
- RT en hydroquinone : 8,5 %
- RT total en diphénols : 30,5 %
- Rapport PC/HQ : 2,6

EXEMPLES 23 à 25

Dans un réacteur en verre pyrex de 30 $cm^3$, muni d'une agitation centrale par barreau aimanté, d'un réfrigérant relié à un gazomètre, d'un système de chauffage régulé et d'un système d'injection, on charge après

avoir préalablement purgé l'appareil à l'aide d'azote :

- 4,7 g de phénol (0,05 mol)
- 0,1 g de $TiO_2$ (IX) ANATASE, calciné 1 heure 30 à 400°C puis 2 heures à 550°C
- 4,7 g de solvant (voir tableau 3 ci-après).

On chauffe à 80°C sous agitation, puis on injecte une solution aqueuse de $H_2O_2$ à 70 % en poids par poids. (0,0025 mol de $H_2O_2$).

On laisse ensuite réagir pendant 2 heures 30 minutes.

Après filtration du catalyseur, on dose $H_2O_2$ non transformée par iodométrie et les diphénols par chromatographie liquide haute performance (CLHP).

Les résultats obtenus sont rassemblés dans le tableau 3 ci-après.

| EXEMPLES | SOLVANTS | TT % H2O2 | RT % PC | RT % HQ | RT % diphénols | Rapport PC/HQ |
|---|---|---|---|---|---|---|
| 23 | Acétonitrile | 97,5 | 40,0 | 11,5 | 51,5 | 3,4 |
| 24 | éthanol | 97,5 | 28,5 | 14,5 | 43,0 | 1,9 |
| 25 | Méthyltertio-butyléther | 95,5 | 42,5 | 11,5 | 54,0 | 3,7 |

TABLEAU 3

EXEMPLES 26 et 27

Dans un réacteur en verre pyrex de 30 cm³, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 9,4 g de phénol (0,10 mol)
- 0,1 g de dioxyde de titane (IX) ANATASE calciné pendant 3 heures à différentes températures.

On chauffe à 80°C sous agitation, puis on injecte une solution aqueuse de $H_2O_2$ à 70 % en poids par poids. (0,0050 mol de $H_2O_2$).

On laisse ensuite réagir pendant 10 minutes à 80°C.

Après filtration du catalyseur, on dose $H_2O_2$ non transformée par iodométrie et les diphénols par chromatographie liquide haute performance (CLHP).

Les résultats obtenus sont rassemblés dans le tableau 4 ci-après :

| EXEMPLES | TEMPERATURE DE CALCINATION | TT % H2O2 | RT % PC | RT % HQ | RT % diphénols | Rapport PC/HQ |
|---|---|---|---|---|---|---|
| 26 | 400°C SBE 122 m2/g * | 99,0 | 42,5 | 10,0 | 52,5 | 4,2 |
| 27 | 700°C SBE 37 m2/g * | 78,5 | 32,0 | 6,5 | 38,5 | 4,9 |

* SBE = surface spécifique BET

TABLEAU 4

EXEMPLES 28 à 30

Dans un réacteur en verre pyrex de 30 cm³, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 9,4 g de phénol (0,10 mol)
- 0,1 ou 0,25 g de dioxyde de titane (IX) ANATASE calciné pendant 3 heures à différentes températures.

On chauffe à 80°C sous agitation, puis on injecte une solution aqueuse de $H_2O_2$ à 70 % en poids par poids. (0,0050 mol de $H_2O_2$).

On laisse ensuite réagir pendant 2 heures 30 à 80°C.

Après filtration du catalyseur, on dose $H_2O_2$ non transformée par iodométrie et les diphénols par chromatographie liquide haute performance (CLHP).

Les résultats obtenus sont rassemblés dans le tableau 5 ci-après :

| EXEMPLES | TEMPERATURE DE CALCINATION | TT % H2O2 | RT % PC | RT % HQ | RT % diphénols | Rapport PC/HQ |
|---|---|---|---|---|---|---|
| 28 * | 700°C<br>SBE 37 m2/g | 92,5 | 23,5 | 7,0 | 30,5 | 3,3 |
| 29 ** | 850°C<br>SBE 7 m2/g | 57,0 | 21,5 | 3,5 | 25,0 | 6,1 |
| 30 ** | 1000°C<br>SBE 2,5 m2/g | 52,0 | 13,5 | 3,0 | 16,5 | 4,5 |

* 0,1 g de $TiO_2$
** 0,25 g de $TiO_2$

TABLEAU 5

EXEMPLES 31 à 35

Dans un réacteur en verre pyrex de 100 cm³, muni d'une agitation centrale, d'un réfrigérant relié à un gazomètre, d'un système de chauffage régulé et d'un système d'injection, on charge après avoir préalablement purgé l'appareil à l'aide d'azote :

- 47 g de phénol (0,50 mol)
- 1 g de dioxyde de titane GEL G5 NC 079 commercialisé par la société THANN ET MULHOUSE, calciné pendant 3 heures à 400°C
- 47 g d'un solvant organique (voir tableau 6 ci-après).

On chauffe à 80°C sous agitation, puis on injecte en 2 mn une solution aqueuse de $H_2O_2$ à 70 % en poids par poids. (0,025 mol de $H_2O_2$).

On laisse ensuite réagir pendant 2 heures à 80°C.

Après filtration du catalyseur, on dose $H_2O_2$ non transformée par iodométrie et les diphénols par chromatographie liquide haute performance (CLHP).

Les résultats obtenus sont rassemblés dans le tableau 6 ci-après :

| EXEMPLES | SOLVANTS | TT % H2O2 | RT % PC | RT % HQ | RT % diphénols | Rapport PC/HQ |
|---|---|---|---|---|---|---|
| 31 | Acétonitrile | 94,6 | 46,3 | 11,3 | 57,6 | 4,1 |
| 32 | Tertiobutanol | 98,5 | 46,8 | 14,8 | 61,6 | 3,2 |
| 33 | Méthyltertio-butyléther | 96,9 | 49,4 | 12,1 | 61,5 | 4,1 |
| 34 | Acétone | 95,3 | 43,2 | 18,5 | 61,7 | 2,3 |
| 35 | Méthylisobutyl-cétone | 97,0 | 42,2 | 10,5 | 52,7 | 4,0 |

TABLEAU 6

## Revendications

1. Procédé d'hydroxylation de phénols ou éthers de phénols de formule générale (I) :

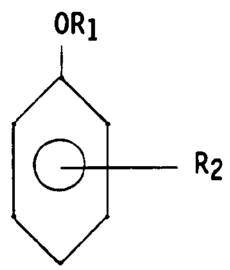

(I)

dans laquelle :
- $R_1$ représente un atome d'hydrogène, un groupement méthyle, un groupement éthyle ou un groupement phényle,
- $R_2$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de dioxyde de titane.

2. Procédé selon la revendication 1, caractérisé en ce qu'il est appliqué aux phénols et éthers de phénol de formule (I) dans laquelle $R_1$ représente un atome d'hydrogène, un groupement méthyle ou un groupement éthyle, et $R_2$ représente un atome d'hydrogène, un groupement méthyle, éthyle ou tertiobutyle, un groupement méthoxy ou éthoxy.

3. Procédé selon l'une des revendications 1 ou 2 caractérisé en ce qu'il est appliqué aux phénols et éthers de phénols choisis parmi le phénol, l'anisole, l'orthocrésol, le métacrésol, le paracrésol, le tertiobutyl-4 phénol, le méthoxy-2 phénol, le méthoxy-4 phénol.

4. Procédé selon l'une des revendications 1 à 3 réalisé en discontinu, caractérisé en ce que le dioxyde de titane représente en poids par rapport au poids du composé de formule (I) engagé de 0,1 % à 20 % et de préférence de 0,5 % à 10 %.

5. Procédé selon l'une des revendications 1 à 3, caractérisé en ce que le procédé est réalisé en continu sur

un lit fixe de dioxyde de titane.

6. Procédé selon l'une des revendications 1 à 5, caractérisé en ce que l'on met en oeuvre le dioxyde de titane anatase, le dioxyde de titane rutile et le dioxyde de titane brookite.

7. Procédé selon l'une des revendications 1 à 6, caractérisé en ce que l'on procède à une calcination du dioxyde de titane pendant une à plusieurs heures à une température de 300°C à 1000°C environ et de préférence de 300°C à 700°C.

8. Procédé selon l'une des revendications 1 à 7, caractérisé en ce que le rapport molaire composé de formule (I)/peroxyde d'hydrogène est de 25/1 à 3/1 et de préférence de 20/1 à 4/1.

9. Procédé selon l'une des revendications 1 à 8, caractérisé en ce que le peroxyde d'hydrogène est mis en oeuvre sous la forme d'une solution aqueuse.

10. Procédé selon l'une des revendications 1 à 8, caractérisé en ce que le peroxyde d'hydrogène est mis en oeuvre sous la forme d'une solution organique.

11. Procédé selon l'une des revendications 1 à 10, caractérisé en ce que la réaction d'hydroxylation est effectuée dans un solvant du composé de formule (I), de préférence miscible ou partiellement miscible à l'eau, tel que l'eau, un alcool, une cétone, un nitrile, un acide carboxylique, un ester, un éther ou un solvant aprotique polaire.

12. Procédé selon l'une des revendications 1 à 11, caractérisé en ce que la réaction d'hydroxylation est conduite à une température comprise entre 45°C et 160°C.

13. Procédé selon l'une des revendications 1 à 11, caractérisé en ce que le dioxyde de titane est mis en oeuvre à l'état de poudre ou de produits mis en forme tels que granulés, billes et monolithes.

**Patentansprüche**

1. Verfahren zur Hydroxylierung von Phenolen oder Phenolethern der allgemeinen Formel (I):

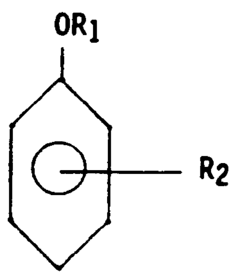

(I)

in der
- $R_1$ ein Wasserstoffatom, eine Methylgruppe, eine Ethylgruppe oder eine Phenylgruppe bedeutet,
- $R_2$ ein Wasserstoffatom, eine Alkylgruppe mit 1 bis 4 Kohlenstoffatomen, eine Alkoxygruppe mit 1 bis 4 Kohlenstoffatomen oder eine Phenyl- oder Cyclohexylgruppe bedeutet, durch Umsetzung mit Wasserstoffperoxid, dadurch gekennzeichnet, daß die Reaktion in Gegenwart einer wirksamen Menge Titandioxid ausgeführt wird.

2. Verfahren nach Anspruch 1, dadurch gekennzeichnet, daß es auf Phenole und Phenolether der Formel (I), in der $R_1$ ein Wasserstoffatom, eine Methylgruppe oder eine Ethylgruppe bedeutet und $R_2$ ein Wasserstoffatom, eine Methyl-, Ethyl- oder tert.Butylgruppe oder eine Methoxy- oder Ethoxygruppe bedeutet, angewandt wird.

3. Verfahren nach einem der Ansprüche 1 oder 2, dadurch gekennzeichnet, daß es auf Phenole und Phenolether angewandt wird, die ausgewählt werden aus Phenol, Anisol, o-Cresol, m-Cresol, p-Cresol, 4-tert.Butylphenol, 2-Methoxyphenol und 4-Methoxyphenol.

4. Verfahren nach einem der Ansprüche 1 bis 3, diskontinuierlich ausgeführt, dadurch gekennzeichnet, daß das Titandioxid, bezogen auf das Gewicht der eingesetzten Verbindung der Formel (I), 0,1 bis 20 Gew.-%, vorzugsweise 0,5 bis 10 Gew.-%, ausmacht.

5. Verfahren nach einem der Ansprüche 1 bis 3, dadurch gekennzeichnet, daß das Verfahren kontinuierlich auf einem Festbett aus Titandioxid ausgeführt wird.

6. Verfahren nach einem der Ansprüche 1 bis 5, dadurch gekennzeichnet, daß man als Titandioxid Anatas, Rutil und/oder Brookit einsetzt.

7. Verfahren nach einem der Ansprüche 1 bis 6, dadurch gekennzeichnet, daß man das Titandioxid während einer bis zu mehreren Stunden bei einer Temperatur von 300 bis etwa 1000°C, vorzugsweise von 300 bis 700°C, brennt.

8. Verfahren nach einem der Ansprüche 1 bis 7, dadurch gekennzeichnet, daß das Molverhältnis von Verbindung der Formel (I)/Wasserstoffperoxid 25/1 bis 2/1, vorzugsweise 20/1 bis 4/1, beträgt.

9. Verfahren nach einem der Ansprüche 1 bis 8, dadurch gekennzeichnet, daß das Wasserstoffperoxid in Form einer wäßrigen Lösung eingesetzt wird.

10. Verfahren nach einem der Ansprüche 1 bis 8, dadurch gekennzeichnet, daß das Wasserstoffperoxid in Form einer organischen Lösung eingesetzt wird.

11. Verfahren nach einem der Ansprüche 1 bis 10, dadurch gekennzeichnet, daß die Hydroxylierungsreaktion in einem Lösungsmittel für die Verbindung der Formel (I) ausgeführt wird, das vorzugsweise mit Wasser mischbar oder teilweise mischbar ist, wie Wasser, ein Alkohol, ein Keton, ein Nitril, eine Carbonsäure, ein Ester, ein Ether oder ein polares aprotisches Lösungsmittel.

12. Verfahren nach einem der Ansprüche 1 bis 11, dadurch gekennzeichnet, daß die Hydroxylierungsreaktion bei einer Temperatur im Bereich von 45 bis 160°C ausgeführt wird.

13. Verfahren nach einem der Ansprüche 1 bis 11, dadurch gekennzeichnet, daß das Titandioxid als Pulver oder als geformtes Produkt, wie Granulat, Kugeln und Monolithe, eingesetzt wird.

## Claims

1. Process for the hydroxylation of phenols or phenol ethers of the general formula (I):

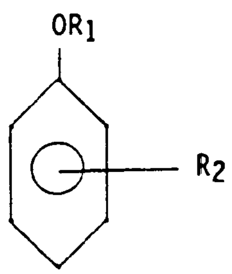

(I)

in which:

$R_1$ represents a hydrogen atom, a methyl group, an ethyl group or a phenyl group, and

$R_2$ represents a hydrogen atom, an alkyl radical having 1 to 4 carbon atoms, an alkoxy radical having 1 to 4 carbon atoms or a phenyl or cyclohexyl radical;

by reaction with hydrogen peroxide, characterised in that the reaction is carried out in the presence of an effective quantity of titanium dioxide.

2. Process according to claim 1, characterised in that it is applied to phenols and phenol ethers of the formula (I) in which $R_1$ represents a hydrogen atom, a methyl group or an ethyl group, and $R_2$ represents a hydrogen atom, a methyl, ethyl or tert-butyl group or a methoxy or ethoxy group.

3. Process according to one of claims 1 or 2, characterised in that it is applied to phenols and phenol ethers selected from phenol, anisole, ortho-cresol, meta-cresol, para-cresol, 4-tert-butylphenol, 2-methoxyphenol and 4-methoxyphenol.

4. Process according to one of claims 1 to 3, carried out discontinuously, characterised in that the titanium dioxide represents 0.1 % to 20 % and preferably 0.5 % to 10 % by weight relative to the weight of the compound of the formula (I).

5. Process according to one of claims 1 to 3, characterised in that the process is carried out continuously over a fixed bed of titanium dioxide.

6. Process according to one of claims 1 to 5, characterised in that anatase titanium dioxide, rutile titanium dioxide and brookite titanium dioxide are used.

7. Process according to one of claims 1 to 6, characterised in that a calcination of the titanium dioxide is carried out for one to several hours at a temperature from about 300°C to 1000°C and preferably 300°C to 700°C.

8. Process according to one of claims 1 to 7, characterised in that the molar ratio compound of the fromula (I)/hydrogen peroxide is 25/1 to 3/1 and preferably 20/1 to 4/1.

9. Process according to one of claims 1 to 8, characterised in that the hydrogen peroxide is used in the form of an aqueous solution.

10. Process according to one of claims 1 to 8, characterised in that the hydrogen peroxide is used in the form of an organic solution.

11. Process according to one of claims 1 to 10, characterised in that the hydroxylation reaction is carried out in a solvent for the compound of the formula (I), which solvent is preferably miscible or partially miscible with water, such as water, an alcohol, a ketone, a nitrile, a carboxylic acid, an ester, an ether or a polar aprotic solvent.

12. Process according to one of claims 1 to 11, characterised in that the hydroxylation reaction is carried out at a temperature between 45°C and 160°C.

13. Process according to one of claims 1 to 11, characterised in that the titanium dioxide is used in the form of a powder or of shaped products such as pellets, spheres or monoliths.